# EUROPEAN PATENT APPLICATION

(11) **EP 2 027 854 A1**
(43) Date of publication of application: **25.02.2009**
(21) Application number: 08168894.7
(22) Date of filing: 18.11.2003
(51) Int. Cl.: A61K 31/135, A61P 29/00

(54) **Analgesic uses of norketamine and ketamine/norketamine prodrugs**

(30) Priority: 18.11.2002 US 426793 P
(62) Divisional of application: 03783623.6
(71) Applicant: Yaupon Therapeutics, Inc., Radnor, PA 19087 (US)
(72) Inventor: Crooks, Peter A., Lexington, FL 40502 (US); Riveral, Miquel, Vradenton, FL 34202 (US)
(74) Representative: Swinkels, Bart Willem

(57) **Abstract**

The present invention relates to norketamine and ketamine/norketamine prodrugs, and methods of their use and analgesics. More particulary, the invention relates to norketamine and N-conjugated prodrugs of ketamine and norketamine, and methods of using these agents for the management of chronic pain without requiring administration of narcotics. The invention relates to self-management of pain on an outpatient basis comprising administering via conventional routes, including transdermal, nasal, rectal, oral, transmucosal, intravenous, intramuscular, and other routes, one or more doses of norketamine and/or ketamine/norketamine prodrugs effective to alleviate pain to a subject suffering from pain.

## Description

### CROSS REFERENCE TO RELATED APPLICATIONS

This application claims priority under 35 U.S.C. § 119(e) to U.S. Provisional Application No. 60/426,793, filed on November 18, 2002, the entire disclosure of which is hereby incorporated herein by reference.

### TECHNICAL FIELD

The present invention relates to norketamine and ketamine/norketamine prodrugs, and methods of use their use as analgesics. More particularly, the invention relates to norketamine and N-conjugated prodrugs of ketamine and norketamine, and methods of using these agents for the management of chronic pain without requiring administration of narcotics. The invention relates to self-management of pain on an outpatient basis comprising administering via conventional routes, including transdermal, nasal, rectal, oral, transmucosal, intravenous, intramuscular, and other routes, of norketamine and/or ketamine/norketamine prodrugs effective to alleviate pain to a subject suffering from pain. Uses of norketamine and ketamine/norketamine prodrugs would also apply, for example, to treating headaches, drug abuse, mood and anxiety disorders, as well as other neuropsychiatric disorders, both motoric and cognitive, such as Alzheimer's disease, Parkinson's syndrome, which are thought to be caused by neurodegeneration.

### BACKGROUND ART

Ketamine (2-(2-chlorophenyl)-2-(methylamino)-cyclohexanone) is a general anesthetic used by anesthesiologists, veterinarians, and researchers. Usually, ketamine is administered intramuscularly (i.m.) or intravenously (i.v.) for induction of anesthesia, though ketamine is considered a versatile anesthetic agent and has been delivered through other, less conventional routes in cases when i.v. routes are unavailable. Nasal administration of ketamine and midazolam to achieve sedation for ophthalmic surgery, and to induce anesthesia prior to elective surgery in healthy children has been reported (Louon et al., 1993, Br. J. Ophthalmol. 77:529-530; Weksler et al., 1993, Can. J. Anaesthesia 40:119-121). Nasal, rectal, and i.v. administrations of ketamine have been compared in association with halothane anesthesia in young children (Malinovsky et al., 1996, Br. J. Anaesthesia 77: 203-207). However, Ketamine is well known to have important clinical disadvantages; particularly, ketamine is known to cause disturbing emergence reactions, including delirium, dysphoria, and unpleasant dreams (Leung et al., 1985, J. Med. Chem. 29: 2396-2399). Current pharmaceutical compositions of ketamine are racemic mixtures of S- and R-ketamine, though S-ketamine has been found recently to be twice as potent as R-ketamine and to allow faster recovery with fewer negative side effects than the racemic mixture (C. S. T. Aun, 1999, Br. J. Anaesthesia 83: 29-41). One of the principal metabolic products of ketamine is norketamine (2-(2-chlorophenyl)-2-amino-cyclohexanone).

Ketamine has also been known also to have analgesic properties (Domino et al., 1965, Clin. Pharmacol. Ther. 6:279); profound analgesia can be achieved with subanesthetic doses of ketamine (Bovill, 1971, Br. J. Anaesth. 43:496; Sadove et al., 1971, Anesth. Analg. 50:452-457). The drug is administered by various routes, including i.v., i.m., caudal, intrathecal, oral, rectal, and subcutaneous (s.c.). Subcutaneous administration of ketamine has been used to treat pain following surgery and associated with terminal cancer (see, e.g., Oshima et al., 1990, Can. J. Anaesth. 37:385-386). Ketamine hydrochloride administered via a subcutaneous cannula was reported to successfully treat phantom limb pain (Stannard and Porter, 1993, Pain 54:227-230). Oral administration of ketamine gave rise to greater concentrations of the norketamine metabolite versus intramuscular administration (Grant et al., 1981, Br. J. Anaesthesia 53: 805-810). These authors also speculate on the analgesic effect of norketamine.

Management of pain, and particularly chronic pain, is complex and frequently unsuccessful. The first line of treatment usually involves administration of µ-opioid agonists, e.g., narcotics such as morphine (see, e.g., Anderson and Brill, 1992, Semin. Anesth. 11:158-171). However, rapid tolerance and marked resistance to narcotics frequently develop, thus rendering these agents ineffective (see, *e.g.,* Abram, 1993, Reg. Anesth. 18(SUPPL):406-413). Non-competitive N-methyl-D-aspartate (NMDA) receptor antagonists, such as ketamine and norketamine, have been reported to interfere with the development of tolerance to the analgesic effects of morphine, possibly through blockade of the NMDA receptor rather than from "side-effects" of the antagonist, since the antagonists were not found to reverse tolerance (Trujillo and Akil, 1994, Brain Res. 633:178-188).

Studies indicate that activation of the NMDA receptor complex in the spinal dorsal horn leads to increased spontaneous neural discharge, expanded receptive fields and exaggerated responses to afferent input. These neural mechanisms may be expressed physically as hyperalgesia (increased pain sensation) and allodynia (pain arising from a stimulus that is not normally painful). Repeated stimuli cause a temporal summation of C-fiber-mediated responses of dorsal horn nociceptive neurons; this phenomenon, increased output to a constant input, is known as "wind-up." Opioids, through their ability to inhibit release of primary afferent neurotransmitters or to inhibit interneurons early in nociceptive pathways, initially reduce or block C-fiber inputs to the deeper dorsal horn nociceptive neurones. However, as the peripheral stimulation continues, wind-up breaks through the input inhibition and the neurons start to respond. Thus at moderate doses, opioids delay the onset of wind-up without inhibiting the process itself. See generally, Chapman, C.R. et al., "Pain Measurement: an Overview", 1985, Pain 22: 1-31; and Hawthorn, Jan et al., "Management of Cancer Pain: International Training for Cancer Nurses", developed by Glaxo Wellcome in collaboration with The International Society of Nurses in Cancer Care, 1996, 6 pp.

Often, pain management involves administration of a plethora of drugs, such as narcotics, agonist-antagonist agents, butorphanols, benzodiazepines, GABA stimulators, barbiturates, barbiturate-like drugs, orally, *e.g.,* in a pill or liquid formulation, or by i.v. or i.m. injection. Opioid agonists and antagonists may be combined. Thus, a combination of drugs can have offsetting effects. More problematic is the possibility of adverse side effects, particularly gastric distress that accompanies oral administration, or the fear that injections can inspire.

Frequently, a patient suffering from chronic pain will require medication to control stomach and other gastric problems as a result of oral administration of drugs. Alternatives to oral self-administration for most of the analgesic and sedative medications for the treatment of chronic pain in addition to perioral administration are not common, can be cumbersome (*e.g.,* i.m., i.v., or s.c. administration requires use of a cannula or needle), and generally require medical training.

U.S. Pat. No. 4,671,953 describes the administration of sedative, analgesic or sedative drugs in a candy matrix, such that the drug enters the bloodstream through the oral mucosal membranes. However, this method suffers from the disadvantage that a sedated patient may fall asleep with the candy remaining in his or her mouth, which can result in choking. Furthermore, because the total dose of the drug in the candy may exceed the desired dose, administration of the candy must be medically supervised. Finally, the candy is simply unsuitable for everyday use, as sucking on a lollipop is an unseemly practice for an employee or business person.

Moreover, when administration is under the control of the patient suffering from pain, *i.e.,* on an outpatient basis, the potential for overdosing or abuse exists, particularly with respect to narcotics.

Studies have shown that ketamine is converted metabolically through demethylation to norketamine, *in vivo,* at rates dependent on the route of administration, with oral and rectal administrations having the fastest rates due to a high degree of first pass metabolism in the liver (see, e.g., Grant et al., 1981, Br. J. Anaesth. 53: 805-810; Grant et al., 1981, Br. J. Anaesth. 55: 1107-1111; Leung et al., 1985, J. Med. Chem. 29: 2396-2399; Malinovsky et al., 1996, Br. J. Anaesthesia 77: 203-207). Norketamine binds the NMDA receptor less tightly than either S- or R-ketamine (Ebert et al., 1997, Eur. J. Pharm. 333: 99-104) and norketamine is speculated to have an anesthetic and analgesic potency one third that of ketamine (C. S. T. Aun, 1999, Br. J. Anaesthesia 83: 29-41), perhaps explaining the absence of administration of norketamine as an analgesic in the prior art.

U.S. Pat. Nos. 5,543,434 and 6,248,789 B1 disclose transmucosal and nasal administrations of ketamine for the management of pain and to reduce drug dependency. Under the methods of Weg, dosages must be kept low in order to avoid the dysphoric side effects attributable to ketamine. However, studies have indicated that norketamine, delivered intravenously (Leung et al., 1985, J. Med. Chem. 29: 2396-2399) or intraspinally (Shimoyama et al., 1999, Pain 81: 85-93) to rats, produced fewer of the adverse sequelae than an equal dose of ketamine.

U.S. Patent No. 6,194,000 B1 discloses the use of NMDA receptor antagonists in a combined immediate release and sustained release form for the treatment of wind-up. However, this patent discloses that treatment by its methods is ineffective to diminish the initial inputs to the pain transmitting nociceptive neurons.

Thus, there is a need in the art for improved methods for management of pain using non-opioid drugs and drugs having reduced side effects.

There is a further need in the art for a rapid method for reducing or eliminating breakthrough pain and pain related to wind-up that is refractory to standard treatment regimens.

There is a further need in the art to avoid oral and injection administration of pain medication.

There is a need in the art for a fast, convenient, and socially acceptable method for patient self-administration of medication to manage or control pain.

There is yet a further need in the art to avoid overdose and abuse of self-administered medication.

These and other needs in the art have been addressed by the instant invention, which is based on the inventor's contention that S-norketamine and R-norketamine, racemic mixtures thereof, and ketamine/norketamine prodrugs, can be used to alleviate pain safely and effectively, in conjunction with or independently of other pain management regimens, and that the aforesaid prodrugs, while inactive or having a reduced activity as delivered, can be converted metabolically to the respective active drug, S-norketamine, R-norketamine, S-ketamine, R-ketamine, (±)ketamine, or (±)norketamine.

The citation or identification of any reference in this application shall not be construed as an admission that such reference is available as prior art to the present invention. However, all references and citations identified in this application are incorporated in their entirety by reference in the present application.

### SUMMARY OF THE INVENTION

The object of the present invention is to provide a drug composition comprising norketamine and/or ketamine/norketamine prodrugs. More specifically, the object of the present invention is to provide a drug composition comprising S-norketamine, or R-norketamine, or (±)norketamine, and/or the prodrugs of S-ketamine, R-ketamine, S-norketamine, R-norketamine, or (±)norketamine provided through the chemical linking of S-ketamine, R-ketamine, S-norketamine, R-norketamine or racemic mixtures to a variety of carboxylic acids and other substituents to afford the formulae shown in Structures 1 and 2. 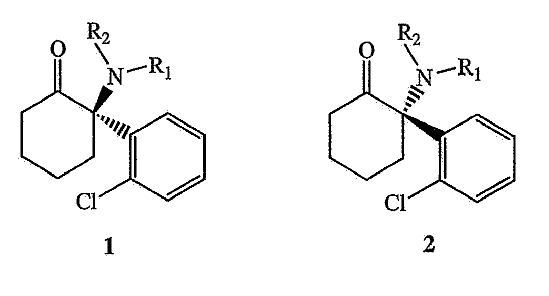 wherein:
R₁ = Methyl, R₂ = CH₂OCOR₃
R₁ = H, R₂ = CH₂OCOR₃
R₁ = Methyl, R₂ = CH₂COOR₃
R₁ = H, R₂ = CH₂COOR₃
R₁ = Methyl, R₂ = COOR₃
R₁ = H, R₂ = COOR₃
R₁ = Methyl, R₂ = COOCH₂CH₂N(CH₃)₂
R₁ = H, R₂ = COOCH₂CH₂N(CH₃)₂
R₁ = Methyl, R₂ = COOCH(R₃)OCOR₄
R₁ = H, R₂ = COOCH(R₃)OCOR₄ 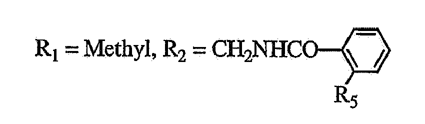 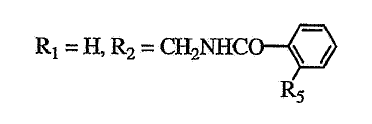 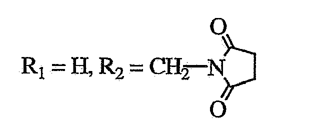 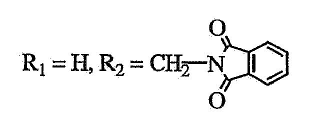 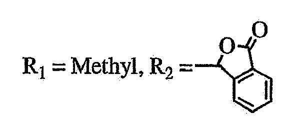 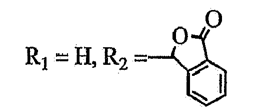 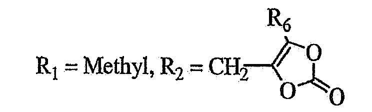 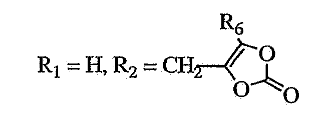
and wherein R₃ and R₄ are phenyl, aryl, azaaryl, alkyl, branched alkyl, cycloalkyl, alkenyl, cycloalkenyl; where R₅ = OH or SH;
and where R₆ = alkyl, branched alkyl;
racemic mixtures of compounds of formula 1 and formula 2 in which R₁ = H and R₂ can be any of the groups recited above, including H; and
pharmaceutically acceptable salts and solvates thereof.

In addition to the prodrugs of the pure enantiomers of ketamine and norketamine (or optically active mixtures of the respective prodrug enantiomers), the present invention also includes racemic mixtures of norketamine, its prodrugs, or prodrugs of ketamine.

The present invention is broadly directed to a method for treating pain in a subject comprising administering one or more doses of norketamine and/or ketamine/norketamine prodrugs effective to alleviate pain to a subject suffering from pain, and compositions for such administration comprising norketamine and/or ketamine/norketamine prodrugs. Compositions of the present invention are delivered by any of a number of routes, including transdermal, nasal, rectal, vaginal, oral, transmucosal, intravenous, intramuscular, caudal, intrathecal, and subcutaneous. In a further embodiment, the present invention provides for pulmonary administration of norketamine and/or ketamine/norketamine prodrugs by inhalation. Transdermal, nasal, and pulmonary administration of an analgesic dose of norketamine and/or ketamine/norketamine prodrugs advantageously allows for patient self administration of the drug, which provides for pain management on an outpatient basis. Moreover, norketamine and/or ketamine/norketamine prodrug administration in transdermal patches, nasal sprays, and inhalers are generally socially acceptable.

Pain therapy on an outpatient basis advantageously reduces the demands on hospital services, results in a substantial decrease in the cost of treatment, and provides the patient with a more normal living and working environment, which can positively affect treatment outcome.

Another advantage of the invention is that it avoids the need to administer narcotic agents for the treatment of chronic pain. Although effective analgesics, narcotics can lose effectiveness due to tolerance or resistance. Narcotics are also highly addictive and subject to abuse.

A further advantage of the invention is that it avoids administration in a candy, which requires medical supervision and is socially questionable, if not outright unacceptable. Transmucosal (including transbuccal, sublingual, vaginal, and rectal), pulmonary, or nasal administration of norketamine or a ketamine/norketamine prodrug will be rapid, allowing for rapid conversion of the prodrug to the parent drug and fast action of the drug, and easily accomplished by a non-medically trained patient. Transdermal administration will be slower acting, but still easily accomplished by a non-medically trained patient. It has further been discovered that effective pain treatment is better achieved by establishing small doses via transdermal or transmucosal delivery, and that these routes of administration avoid side effects associated with a bolus dose of ketamine delivered via i.v. or i.m. administration. Furthermore, a small transmucosal or transdermal dose of norketamine and/or ketamine/norketamine prodrugs can be administered more frequently, which achieves a virtual steady state level of the drug that can be modulated as needed by the subject. Where a patient's condition prevents nasal administration of norketamine and/or ketamine/norketamine prodrugs, ocular administration, using, *e.g.,* norketamine and/or ketamine/norketamine prodrugs drops, can be substituted. In addition to transmucosal route of administration, e.g., nasal, transbuccal, sublingual, vaginal, and rectal, the invention contemplates oral administration (via the gastrointestinal tract, rather than the oral-pharyngeal mucosa), and parenteral administration, e.g., intravenous, intraarterial, intraperitoneal, intradermal, intramuscular, intraventricular, or subcutaneous.

In one aspect, the pain-alleviating dose of norketamine and/or ketamine/norketamine prodrugs is about 0.01 to about 20 mg/kg of body weight. In a more preferred aspect, the dose of norketamine and/or ketamine/norketamine prodrugs is about 0.05 to about 8 mg/kg of body weight. In another embodiment, the total dose of norketamine and/or ketamine/norketamine prodrugs per administration ranges from about 1 to about 30 mg.

In a specific aspect of the invention, the dose of norketamine and/or ketamine/norketamine prodrugs is effective to alleviate breakthrough pain or pain related to wind-up in a patient suffering from a chronic pain condition or from neuropathic pain.

In another specific aspect of the invention, the dose of norketamine and/or ketamine/norketamine prodrugs is effective to alleviate breakthrough pain associated with labor, particularly transition labor.

In a particular aspect, oral, transdermal, transmucosal, pulmonary, or nasal administration of norketamine and/or ketamine/norketamine prodrugs can be a supplemental therapy in a pain management regimen that include administration of one or more of narcotics, analgesics, and sedatives, *e.g*., as described above. In each case, the second pain medication can be administered via the same route as norketamine and/or ketamine/norketamine prodrugs, where appropriate, or via a different route, *e.g.,* orally while the norketamine and/or ketamine/norketamine prodrug is administered transdermally or transbuccally. It has been a surprising discovery in the context of the present invention that norketamine and/or ketamine/norketamine prodrug administration to manage pain on top of an ongoing pain management regimen involving other medications, notably narcotics, allows for reduction over time of the other analgesic, particularly a narcotic analgesic. Generally, experience shows that the effects of different analgesics are additive, and additional analgesics are added to a dosage regimen to supplement it, e.g., when tolerance to a first analgesic builds up. Administration of norketamine and/or ketamine/norketamine prodrugs by methods of the present invention avoids this tolerance build-up.

The present invention further contemplates administering a dose of a benzodiazepine effective to inhibit the dysphoria that can be associated with administration of high doses of norketamine and/or ketamine/norketamine prodrugs. In a preferred aspect, the benzodiazepine is administered simultaneously with the norketamine and/or ketamine/norketamine prodrug via the same route of administration. It should be noted, however, that a further advantage of the instant invention is that it avoids dosing a patient with dysphoric or hallucinogenic amounts of norketamine and/or ketamine/norketamine prodrugs by providing a metered, analgesic dose which is well below the level associated with dysphoria or hallucination.

In yet a further embodiment, the present invention contemplates administering a dose of a narcotic analgesic effective to alleviate pain with the norketamine and/or ketamine/norketamine prodrugs; preferably the narcotic analgesic is administered simultanously with the norketamine and/or ketamine/norketamine prodrug via the same route of administration.

Accordingly, the invention provides a device for patient self-administration of norketamine and/or ketamine/norketamine prodrugs. In its broadest aspect, the device of the invention comprises a pulmonary inhaler containing a formulation of norketamine and/or ketamine/norketamine prodrugs, optionally with a pharmaceutically acceptable dispersant, wherein the device is metered to disperse an amount of the formulation that contains a dose of norketamine and/or ketamine/norketamine prodrug effective to alleviate pain. The dispersant may be a surfactant, such as, but not limited to, polyoxyethylene fatty acid esters, polyoxyethylene fatty acid alcohols, and polyeoxyethylene sorbitan fatty acid esters.

In one specific embodiment, the formulation is a dry powder formulation in which the norketamine and/or ketamine/norketamine prodrug is present as a finely divided powder. The dry powder formulation can further comprise a bulking agent, such as, but not limited to, lactose, sorbitol, sucrose and mannitol, or the norketamine and/or ketamine/norketamine prodrugs may be associated with carrier particles.

In another specific embodiment, the formulation is a liquid formulation, optionally comprising a pharmaceutically acceptable diluent, such as, but not limited to, sterile water, saline, buffered saline and dextrose solution.

In further embodiments, the formulation further comprises a benzodiazepine in a concentration such that the metered amount of the formulation dispersed by the device contains a dose of the benzodiazepine effective to inhibit dysphoria, or a narcotic in a concentration such that the metered amount of the formulation dispersed by the device contains a dose of the narcotic effective to alleviate pain. The present invention further contemplates including both a benzodiazepine and a narcotic in the formulation.

Thus, it is an object of the invention to provide for self administration of a safe, non-narcotic drug for outpatient treatment of pain.

It is a further object of the present invention to provide a method administration of the drug in a controlled amount for the treatment of pain.

Accordingly, the invention provides various pharmaceutical carriers for patient self-administration of norketamine and ketamine/norketamine prodrugs. Examples of such carriers include, but are not limited to, a suppository, a gum, a candy, or a lozenge; other carriers include a transbuccal patch and a transdermal patch. Preferably, the formulation is a sustained-release formulation.

Yet a further object of the invention is to provide a device that can be used outside a hospital or medical office by non-medical personnel for self administration of norketamine and/or ketamine/norketamine prodrugs.

These and other objects of the present invention will become more readily apparent by reference to the following detailed description.

### DETAILED DESCRIPTION OF THE INVENTION

In its primary aspect, the present invention relates to the administration of norketamine and/or ketamine/norketamine prodrugs for the treatment of pain. In a more preferred aspect, the invention provides a method and device for patient self administration of norketamine and/or ketamine/norketamine prodrugs for pain management.

(±)Norketamine and prodrugs of ketamine and norketamine of the present invention are described by formulae 1 and 2, as follows: 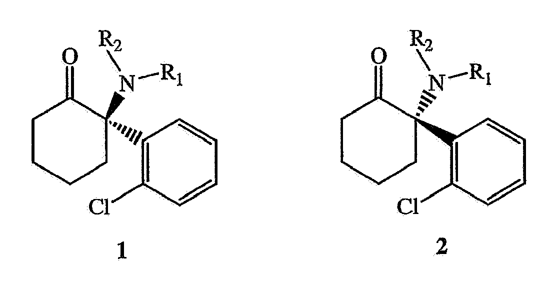 wherein:
R₁ = Methyl, R₂ = CH₂OCOR₃
R₁ = H, R₂ = CH₂OCOR₃
R₁ = Methyl, R₂ = CH₂COOR₃
R₁ = H, R₂ = CH₂COOR₃
R₁ = Methyl, R₂ = COOR₃
R₁ = H, R₂ = COOR₃
R₁ = Methyl, R₂ = COOCH₂CH₂N(CH₃)₂
R₁ = H, R₂ = COOCH₂CH₂N(CH₃)₂
R₁ = Methyl, R₂ = COOCH(R₃)OCOR₄
R₁ = H, R₂ = COOCH(R₃)OCOR₄ 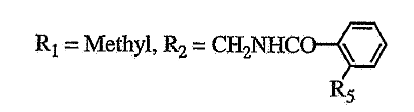 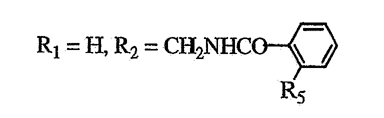 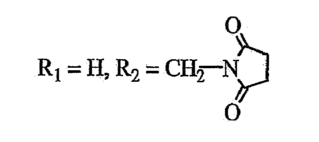 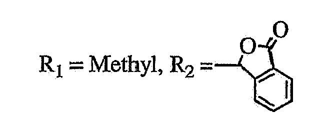 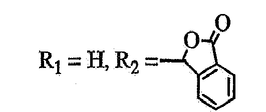 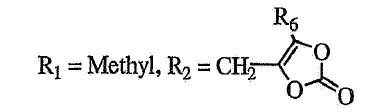 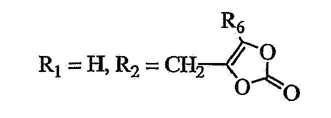 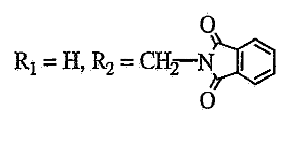
and wherein R₃ and R₄ are phenyl, aryl, azaaryl, alkyl, branched alkyl, cycloalkyl, alkenyl, cycloalkenyl;
where R₅ = OH or SH;
and where R₆ = alkyl, branched alkyl;
racemic mixtures of compounds of formula 1 and formula 2 in which R₁ = H and R₂ can be any of the groups recited above, including H; and
pharmaceutically acceptable salts and solvates thereof.

The invention can alleviate pain from many causes, including but not limited to shock; limb amputation; severe chemical or thermal burn injury; sprains, ligament tears, fractures, wounds and other tissue injuries; dental surgery, procedures and maladies; labor and delivery; during physical therapy; post operative pain; radiation poisoning; cancer; acquired immunodeficiency syndrome (AIDS); epidural (or peridural) fibrosis; failed back surgery and failed laminectomy; sciatica; painful sickle cell crisis; arthritis; autoimmune disease; intractable bladder pain; and the like. Administration of norketamine or a ketamine/norketamine prodrug is also amenable to hospice use, particularly hospices that specialize in the care of cancer and AIDS patients.

In one embodiment, administration of norketamine and/or ketamine/norketamine prodrugs can relieve or alleviate episodes of acute breakthrough pain or pain related to wind-up that can occur in a chronic pain condition. In a further embodiment, administration of norketamine and/or ketamine/norketamine prodrugs can be used as an adjunct therapy to a conventional treatment regimen for a chronic pain condition to alleviate breakthrough pain or pain related to wind-up.

A particular advantage of the present invention for reducing labor and delivery pain is that norketamine and ketamine/norketamine prodrugs in low doses are not known to have significant adverse effects on the fetus.

In a related embodiment, transdermal administration of norketamine and/or ketamine/norketamine prodrugs can be used as an adjunct or directly to treat an acute asthma attack. Since unrelated pain conditions can induce asthma, the present invention advantageously provides for alleviating pain, thus blocking the cause of the attack. Furthermore, ketamine and norketamine (in contrast to narcotic pain medications) are bronchodilators.

In yet another related embodiment, administration of norketamine and/or ketamine/norketamine prodrugs can be used in the treatment of acute nausea. Transdermal, transmucosal, or nasal norketamine and/or ketamine/norketamine prodrugs are particularly attractive for this condition, as nausea precludes the use of oral medications. In particular, a transdermal, transmucosal, or nasal administration can alleviate pain that may be causing the nausea, and can alleviate the abdominal pain that frequently accompanies sever nausea.

In yet a further related embodiment, administration of norketamine and ketamine/norketamine prodrugs can be used to treat acute agitation, for example, agitation exhibited by an alcohol or drug intoxicated individual, or by a person placed under arrest by the police.

Similarly, administration of norketamine and/or ketamine/norketamine prodrugs may be useful in the treatment of shock resulting from severe injuries. Thus, even if a patient fails to sense pain because of severe shock, the extreme pain associated with a severe injury contributes to shock.

The present invention is based on the surprising and unexpected discovery that administration of norketamine and/or ketamine/norketamine prodrugs can alleviate symptoms of chronic pain or neuropathic pain. Thus, a patient suffering from intractable bladder pain, and taking a variety of narcotics, analgesics, and sedatives in an unsuccessful attempt to control the pain, will be able to achieve more satisfactory pain management by administration of 16-32 mg of norketamine and/or ketamine/norketamine prodrugs, corresponding to 0.26 to 0.53 mg/kg of body weight. The dosage is effective for about 15 minutes to about 1 hour in alleviating pain. The patient is able to reduce the amount of a oral pain medications, which often cause gastric distress.

The norketamine and/or ketamine/norketamine prodrugs will preferably be prepared in a formulation or pharmaceutical composition appropriate for administration by the transmucosal route, e.g., nasal, transbuccal, sublingual, vaginal, and rectal; by the oral route (via the gastrointestinal tract, rather than the oral-pharyngeal mucosa); by the pulmonary route (*i.e.,* inhaled); or by the parenteral route, *e.g.,* intravenous, intraarterial, intraperitoneal, intradermal, intramuscular, intraventricular, or subcutaneous. Suitable formulations are discussed in detail, infra. In a further embodiment, the norketamine or a ketamine/norketamine prodrug can be formulated with a mucosal penetration enhancer to facilitate delivery of the drug. The formulation can also be prepared with pH optimized for solubility, drug stability, absorption through skin or mucosa, and other considerations.

The invention provides for administration of a therapeutically effective dose of norketamine and/or ketamine/norketamine prodrugs, *i.e.,* a dose effective to alleviate pain. The actual dose will vary, depending on the body weight of the patient, the severity of the pain, the route of administration, the nature of medications administered concurrently, the number of doses to be administered per day, and other factors generally considered by the ordinary skilled physician in the administration of drugs. In a specific embodiment, the amount of norketamine and/or ketamine/norketamine prodrugs administered to a patient suffering from chronic pain is about 1 to about 50% of the amount used to induce anesthesia; more preferably about 5% to about 40%, and even more preferably about 10% to about 20%.

In another specific embodiment, the dose of norketamine and/or ketamine/norketamine prodrugs is about 0.01 mg per kg of body weight (0.01 mg/kg) to about 20 mg/kg; preferably about 0.05 mg/kg to about 8 mg/kg. In yet another embodiment, the dose ranges from about 1 mg to about 30 mg. Preferably, the effective dose is titrated under the supervision of a physician or medical care provider so that the optimum dose for the particular application is accurately determined. Thus, the present invention provides a dose suited to each individual patient.

Once the dosage range is established, a further advantage of the invention is that the patient can administer the norketamine and/or ketamine/norketamine prodrugs on an as-needed, dose-to-effect basis. Thus, the frequency of administration is under control of the patient. However, the relatively low dose with each administration will reduce the possibilities for abuse that arise under patient self-administration.

Yet another particular advantage of the present invention is that transmucosal or pulmonary administration of the norketamine and/or ketamine/norketamine prodrugs is non-invasive, and provides for introduction into the bloodstream almost as fast as i.v. administration, and much faster than perioral administration.

More importantly, a patient can control administration of the pain medication, because transmucosal or pulmonary administration provides for precise control over the dosage and effect of the drug used to offset changes in activity and pain levels throughout a day. Transmucosal or pulmonary administration of the norketamine and/or ketamine/norketamine prodrugs optimally provides for dose-to-effect administration of the drug. Transdermal administration, though not as fast acting, similarly allows for precise control of the dosage and also provides for excellent dose-to-effect administration of the drug.

Thus, according to the invention, the patient can safely administer an amount of drug effective to alleviate pain by controlling the amount and frequency of administration of a formulation according to the invention. Safe patient regulated control of pain medication is an important advantage because pain is such a subjective condition. The advantage is two-fold here, as the patient can effectively alleviate pain, and the power to alleviate the pain will have significant psychological benefits. A positive psychological attitude can significantly improve the course and outcome of a treatment regimen, as well as making the entire process more bearable to the patient.

Various terms are used throughout the specification, which are defined herein:

As can be readily appreciated by one of skill in the art, the term "ketamine" refers to ketamine (2-o-chlorophenyl)-2-methylamino-cyclohexanone, pharmaceutically acceptable salts thereof, and biologically equivalent derivatives and analogs thereof, e.g., ketamine aspartate, ketamine succinate, etc. In specific embodiments, ketamine refers to salts of ketamine, such as ketamine hydrochloride. There is no limitation on the nature of these salts, provided that, when used for therapeutic purposes, they are pharmaceutically acceptable, which, as is well-known in the art, means that they do not have reduced activity or increased toxicity compared with the free compounds. Examples of these salts include: salts with an inorganic acid such as hydrochloric acid, hydrobromic acid, hydriodic acid, nitric acid, perchloric acid, sulfuric acid or phosphoric acid; and salts with an organic acid, such as methanesulfonic acid, trifluoromethanesulfonic acid, ethanesulfonic acid, benzenesulfonic acid, p-toluenesulfonic acid, fumaric acid, oxalic acid, maleic acid, citric acid, succinic acid, tartaric acid; and other mineral and carboxylic acids well known to those skilled in the art. Examples of salts with inorganic cations such as sodium, potassium, calcium, magnesium, lithium, aluminum, zinc, etc; and salts formed with pharmaceutically acceptable amines such as ammonia, alkylamines, hydroxyalkylamines, lysine, arginine, N-methylglucamine, procaine and the like. Also included within the scope of the term "ketamine," as one of ordinary skill would presume, are isomers and enantiomers thereof that demonstrate analgesic properties, *e.g.,* with greater potency or fewer side effects, or both, and racemic or optically active mixtures of S- and R- ketamine in any combination.

As can be readily appreciated by one of skill in the art, the term "norketamine" refers to norketamine (2-o-chlorophenyl)-2-amino-cyclohexanone, pharmaceutically acceptable salts thereof, and biologically equivalent derivatives and analogs thereof, *e.g.,* norketamine aspartate, norketamine succinate, etc. In specific embodiments, ketamine, norketamine refers to salts of norketamine, such as norketamine hydrochloride. There is no limitation on the nature of these salts, provided that, when used for therapeutic purposes, they are pharmaceutically acceptable, which, as is well-known in the art, means that they do not have reduced activity or increased toxicity compared with the free compounds. Examples of these salts include:
salts with an inorganic acid such as hydrochloric acid, hydrobromic acid, hydriodic acid, nitric acid, perchloric acid, sulfuric acid or phosphoric acid; and
salts with an organic acid, such as methanesulfonic acid, trifluoromethanesulfonic acid, ethanesulfonic acid, benzenesulfonic acid, p-toluenesulfonic acid, fumaric acid, oxalic acid, maleic acid, citric acid, succinic acid, tartaric acid; and other mineral and carboxylic acids well known to those skilled in the art. Examples of salts with inorganic cations such as sodium, potassium, calcium, magnesium, lithium, aluminum, zinc, etc; and salts formed with pharmaceutically acceptable amines such as ammonia, alkylamines, hydroxyalkylamines, lysine, arginine, N-methylglucamine, procaine and the like. Also included within the scope of the term "norketamine," as one of ordinary skill would presume, are isomers and enantiomers thereof that demonstrate analgesic properties, e.g., with greater potency or fewer side effects, or both, and racemic and optically active mixtures of S- and R- norketamine in any combination.

"Prodrugs of norketamine" is used herein to refer to all compounds that may be converted physiologically to norketamine. While it is well known that ketamine is metabolized to norketamine *in vivo,* it is important to note that ketamine is not to be considered a prodrug of norketamine, and the term "norketamine prodrug" in all its forms specifically excludes ketamine as used in this application.

The term "breakthrough pain" is used herein in accordance with its usual meaning in pain treatment. For example, breakthrough pain can refer to pain experienced by a subject receiving treatment for pain, but who experiences a level of pain that is not treatable by the current treatment regimen. "Spike pain" is an acute form of breakthrough pain. Usually medications or therapies for chronic pain do not provide adequate relief for breakthrough pain, either because the maximum pain relief effects of these regimens have been achieved, because of tolerance to medications that has developed, or because the treatment is not fast enough. Pain related to "wind up" is that pain arising from repeated stimuli which causes a temporal summation of C-fiber-mediated responses of dorsal horn nociceptive neurons and that may be expressed physically as hyperalgesia (increased pain sensation) and allodynia (pain arising from a stimulus that is not normally painful).

A subject in whom administration of norketamine and/or ketamine/norketamine prodrugs is an effective therapeutic regimen for management of pain, or for synergism with alternative pain therapy is preferably a human, but can be any animal. Thus, as can be readily appreciated by one of ordinary skill in the art, the methods and devices of the present invention are particularly suited to administration of norketamine and/or ketamine/norketamine prodrugs to any animal, particularly a mammal, and including, but by no means limited to, domestic animals, such as feline or canine subjects, farm animals, such as but not limited to bovine, equine, caprine, ovine, and porcine subjects, wild animals (whether in the wild or in a zoological garden), research animals, such as mice, rats, rabbits, goats, sheep, pigs, dogs, cats, etc., *i.e.,* for veterinary medical use. For veterinary use, rectal administration or transdermal administration are convenient and allow for minimal aggravation or irritation of the animal.

The term "mucosal" refers to a tissue comprising a mucous membranes, such as the oral, buccal, rectal, or vaginal mucosa and the pulmonary mucosa. "Transmucosal" refers to administration of a drug through the mucosa to the bloodstream for systemic delivery of the drug. One distinct advantage of transmucosal delivery is that it provides delivery of drug into the bloodstream almost as fast as parenteral delivery, but without the unpleasant necessity of injection.

The term "transdermal administration" in all its grammatical forms refers to administration of a drug through the dermis to the bloodstream for systemic delivery of the drug. The advantages of transdermal administration for drug delivery are that it does not require injection using a syringe and needle, it avoids necrosis that can accompany i.m. administration of drugs, it avoids the need to constantly suck on a lollipop, and transdermal administration of a drug is highly amenable to self administration.

"Pulmonary administration" refers to administration of a drug through the pulmonary tract (*i.e.,* inhaled into the lungs) to the bloodstream for systemic delivery of the drug. The present invention contemplates pulmonary administration through an inhaler in a particular aspect.

The term "mucosal penetration enhancer" refers to a reagent that increases the rate or facility of transmucosal penetration of norketamine or a ketamine/norketamine prodrug, such as but not limited to, a bile salt, fatty acid, surfactant or alcohol. In specific embodiments, the permeation enhancer can be sodium cholate, sodium dodecyl sulphate, sodium deoxycholate, taurodeoxycholate, sodium glycocholate, dimethylsulfoxide or ethanol.

A "therapeutically effective amount" of a drug is an amount effective to demonstrate a desired activity of the drug. According to the instant invention, a therapeutically effective amount of norketamine or a ketamine/norketamine prodrug is an amount effective to alleviate, *i.e.,* noticeably reduce, pain in a patient.

The invention will now be described in greater detail, with particular reference to transdermal, transmucosal, and pulmonary administration of the norketamine and/or ketamine/norketamine prodrugs and additional therapeutically active drugs or agents with which the norketamine and/or ketamine/norketamine prodrugs can be administered.

### Synthesis of Ketamine/Norketamine Prodrugs

Several new alkyloxy, aryloxycarbonyl and alkylacetyloxy norketamine derivatives have been prepared according to a modification of the procedure reported by Leung *et al* (Louis Y. Leung and Thomas A. Ballie, Camparative Pharmacology in the Rat of Ketamine and Its Two Principal Metabolites, Norketamine and (Z)-6-Hydroxynorketamine. Journal of Medicinal Chemistry (1986), 29(11), 2396-2399).

### 1. N-Alkyloxycarbonyl norketamines

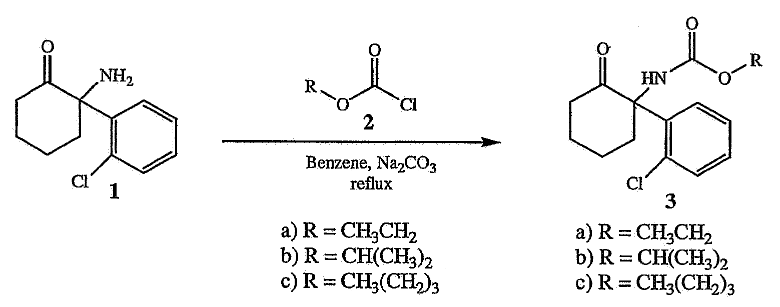

### 1.1 [1-(2-Chloro-phenyl)-2-oxo-cyclohexyl]-carbamic acid ethyl ester (3a)

To a mixture of **1** (224 mg, 1.0 mmol) in anhydrous benzene (8 ml) and Na₂CO₃ (366 mg) was added a solution of ethyl chloroformate (287 mg, 2.66 mmol) in anhydrous benzene (0.5 ml). After heating under reflux for 6 h, the reaction mixture was cooled to room temperature and washed with H₂O, 10% Na₂CO₃, H₂O. The organic layer was diluted with diethyl ether, dried over anhydrous MgSO₄, filtered and evaporated. After recrystallized from diethyl ether, 3a was obtained as a white solid, yield 282 mg (95%): mp. 112-114 °C;
¹H-NMR (CDCl₃) 6.7-7.6 (m, 4H, Ar H), 4.03 (m, 2H, OCH₂CH₃), 1.4-2.8 (m, cyclohexanone CH₂ protons), 1.26 (*t*, 3H, OCH₂CH₃); MS, *mlz* 295 (M⁺, 2%), 260 ([M-Cl]⁺, 20%), 232 ([M-Cl-CO]⁺, 100%).

### 1.2[1-(2-Chloro-phenyl)-2-oxo-cyclohexyl]-carbamic acid isopropyl ester (3b)

To a mixture of 1 (224 mg, 1.0 mmol) in anhydrous benzene (8 ml) and Na₂CO₃ (366 mg) was added a solution of isopropyl chloroformate (326 mg, 2.66 mmol) in anhydrous benzene (0.5 ml). After heating under reflux for 3 h, the reaction mixture was cooled to room temperature and filtered. The mixture was diluted with diethyl ether and evaporated. After recrystallization from diethyl ether, 3b was obtained as a white solid, yield 298 mg (96%): mp. 124-126°C; ¹H-NMR (CDCl₃) 6.8-7.6 (m, 4H, Ar H), 4.16 (m, 1H, OCH[CH₃)₂]), 1.6-2.6 (m, cyclohexanone CH₂ protons), 1.36 (d, 6H, OCH[CH₃)₂]); MS, *m*/*z* 309 (M⁺, 2%), 274 ([M-Cl]⁺, 12%), 246 ([M-Cl-CO]⁺, 65%), 204 (100%).

### 1.3[1-(2-Chloro-phenyl)-2-oxo-cyclohexyl]-carbamic acid butyl ester (3c)

To a mixture of 1 (224 mg, 1.0 mmol) in anhydrous benzene (8 ml) and Na₂CO₃ (366 mg) was added a solution of butyl chloroformate (363 mg, 2.66 mmol) in anhydrous benzene (0.5 ml). After heating under reflux for 2 h, the reaction mixture was cooled to room temperature and filtered. The mixture was diluted with diethyl ether and evaporated. After recrystallization from diethyl ether, **3c** was obtained as a white solid, yield 308 mg (95%): mp. 130-132 °C, ¹H-NMR (CDCl₃) 7.0-7.4 (m, 4H, Ar H), 4.08 (m, 2H, OCH₂CH₂CH₂CH₃), 1.7-2.6 (m, cyclohexanone CH₂ protons), 1.3-1.6 (m, 4H, OCH₂CH₂CH₂CH₃), 1.0 (t, 3H, OCH₂CH₂CH₂CH₃); MS, m/z323 (M⁺, 1%), 288 ([M-Cl]⁺, 14%), 260 ([M-Cl-CO]⁺, 100%).

### 2. N-Aryloxycarbonyl norketamines

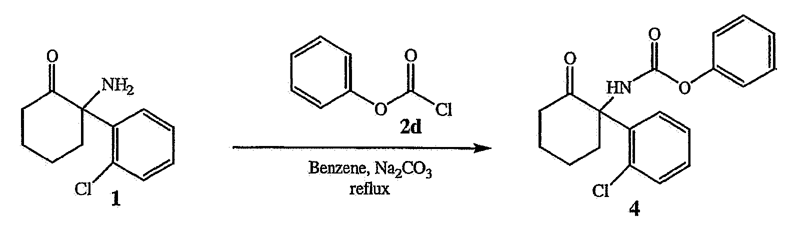

### 2.1[1-(2-Chloro-phenyl)-2-oxo-cyclohexyl]-carbamic acid phenyl ester (4)

To a mixture of **1** (224 mg, 1.0 mmol) in anhydrous benzene (8 ml) and Na₂CO₃ (366 mg) was added a solution of phenyl chloroformate (448 mg, 2.86 mmol) in anhydrous benzene (0.5 ml). After heating under reflux for 8 h, the reaction mixture was cooled to room temperature and filtered. The mixture was diluted with diethyl ether and evaporated. After recrystallization from diethyl ether, **4** was obtained as a white solid, yield 275 mg (80%): mp. 137-139 °C, ¹H-NMR (CDCl₃) 6.9-7.6 (m, 9H, Ar H), 1.6-2.6 (m, cyclohexanone CH₂ protons); MS, *m*/*z* 341 (M⁺-2, 1%), 281 ([M-Cl-CO]⁺, 100%).

### 3. N-Benzyloxycarbonyl norketamines

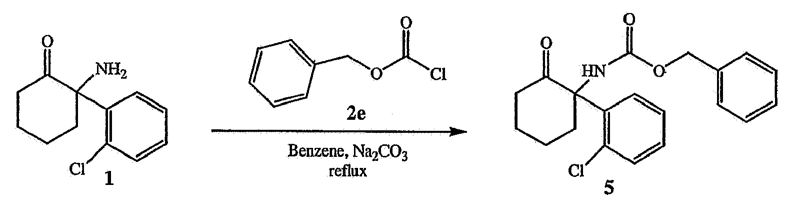

### 3.1[1-(2-Chloro-phenyl)-2-oxo-cyclohexyl]-carbamic acid benzyl ester (5)

To a mixture of **1** (224 mg, 1.0 mmol) in anhydrous benzene (8 ml) and Na₂CO₃ (366 mg) was added a solution of benzyl chloroformate (488 mg, 2.86 mmol) in anhydrous benzene (0.5 ml). After heating under reflux for 6 h, the reaction mixture was cooled to room temperature and filtered. The mixture was diluted with diethyl ether and evaporated. After recrystallization from diethyl ether, **5** was obtained as a white solid, yield 258 mg (72%): MS, m/z 357 (M⁺, 0.5%),322 ([M-Cl]⁺, 8%), 294 ([M-Cl-CO]⁺, 26%).

### 4. N-Alkylacetate norketamines

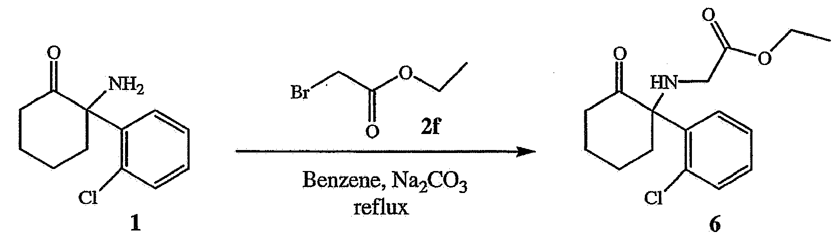

### 4.1 [1-(2-Chloro-phenyl)-2-oxo-cyclohexylamino]-acetic acid ethyl ester (6)

To a mixture of **1** (224 mg, 1.0 mmol) in anhydrous benzene (8 ml) and Na₂CO₃ (366 mg) was added a solution of ethyl bromoacetate (478 mg, 2.66 mmol) in anhydrous benzene (0.5 ml). After heating under reflux for 36 h, the reaction mixture was cooled to room temperature and filtered. The mixture was diluted with diethyl ether and evaporated. After recrystallization from diethyl ether, 6 was obtained as a white solid, yield 258 mg (92%): MS, *m*/*z* 309 (M⁺, 1%), 281 ([M-CO]⁺, 23%).

### 5. N-Acyloxyalkylketamines and N-acyloxyalkylnorketamines

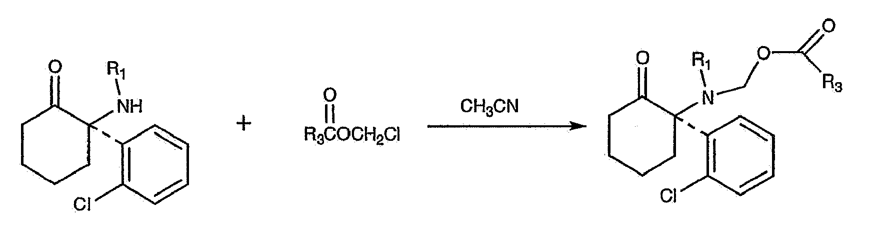

R₁=H or CH₃,
R₃=phenyl, aryl, azaaryl, alkyl, branched alkyl, cycloalkyl, alkenyl, cycloalkenyl

Chloromethyl esters of different acids can generally be easily made from the corresponding acyl chloride and paraformaldehyde under high temperature conditions (Chen *et al,* 2003). The purified products react with either ketamine or norketamine under normal conditions to give the appropriate novel derivative.

### 6. N-(Acetic acid esters) of ketamine and norketamine

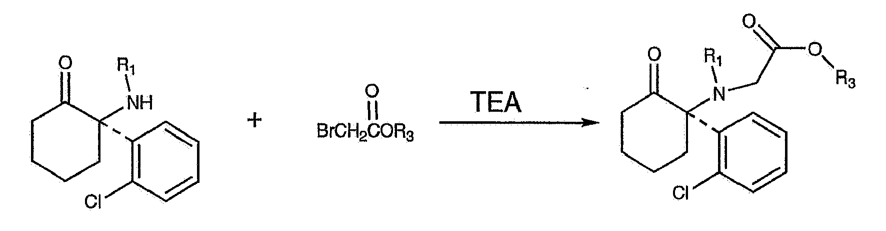

R₁=H or CH₃,
R₃=phenyl, aryl, azaaryl, alkyl, branched alkyl, cycloalkyl, alkenyl, cycloalkenyl

Different O-alkyl bromo- or chlor-acetates are commercially available. Under basic conditions, they react with primary or secondary amines to form secondary or tertiary amines (Soede-Huijbregts, *et al,* 2001). Employing this methodology, the corresponding ketamine and norketamine N-acetic acid esters can be prepared.

### 7. Aminoalkyloxycarbonylketamines and aminoalkyloxycarbonylnorketamines

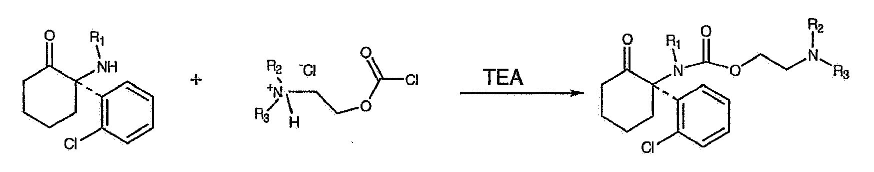

R₁==H or CH₃

Aminoalkyloxycarbonylamine prodrugs will behave similarly to the alkyloxycarbonylamine prodrugs, which are easily hydrolyzed to the parent amine drug *in vivo.* The preparation of these prodrugs is achieved according to the general reaction shown above (Boyle, *et al,* 1997). The 2-aminoethyl chloroformate reactant is made from 2-dimethylaminoethanol and phosgene under normal conditions as shown below (Brotherton and Lynn, 1961). This intermediate can then be reacted with either ketamine or norketamine to afford the corresponding aminoalkyloxycarbonyl prodrug

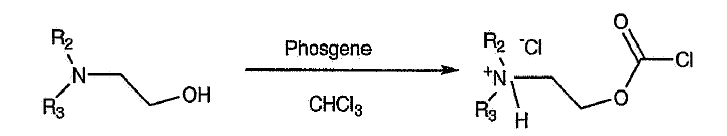

### 8. Acyloxyalkoxycarbonylketamines and acyloxyalkoxycarbonylketamines norketamines

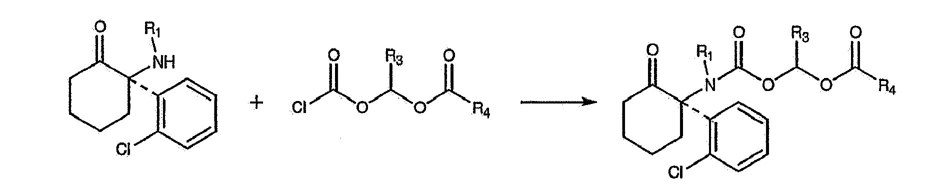

R₁=H or CH₃,
R₃=phenyl, aryl, azaaryl, alkyl, branched alkyl, cycloalkyl, alkenyl, cycloalkenyl
R4=phenyl, aryl, azaaryl, alkyl, branched alkyl, cycloalkyl, alkenyl, cycloalkenyl

This type of prodrug is more easily hydrolyzed to the parent amine drug than other prodrugs because of the multi-reactive points in the structure. The α,α-alkene diester intermediates are prepared from α -chloroalkyl chloroformate (commercially available) through the steps outlined below (Lund, 1991). Then, the resulting chloroformate intermediate is reacted with either ketamine or norketamine to give the desired acyloxyalkoxycarbonyl prodrug (Lund, 1991).

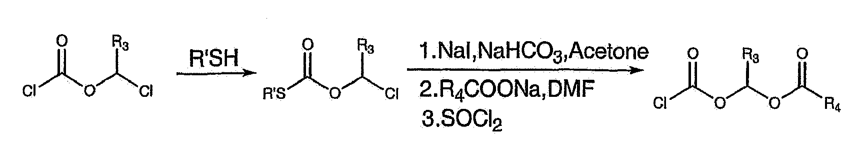

### 9. N-(Arylamidemethylene)ketamines and N-(Arylamidemethylene)norketamines

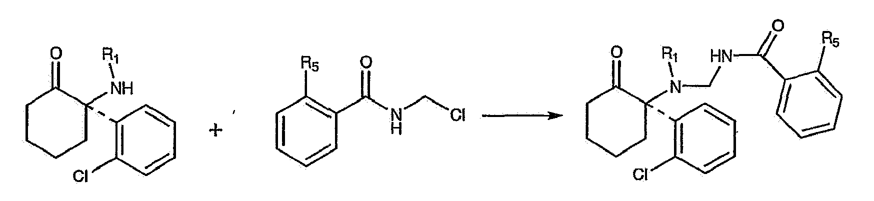

R₁=H or CH₃,
R₅=OH or SH

The reactions between ketamine or norketamine and N-(chloromethyl)benzamide derivatives yields another type of ketamine prodrug (Fischer, *et al,* 1985). The intermediate N-(chloromethyl)benzamide) can also be benzene ring substituted to afford a series of N-(arylamidemethylene) prodrugs. These prodrugs are obtained from the corresponding benzamides and paraformaldehyde followed by chlorination of the hydroxyl derivatives formed (Fischer, *et al,* 1985) and reaction of the resulting N-(chloromethyl)benzamide with either ketamine or norketamine.

### 10. N-(N'-phthalimidomethylene)ketamine, N-(N'-phthalimidomethylene)norketamine, N-(N'-2,5-pyrrolidinedionemethylene)ketamine and N-(N'- 2,5-pyrrolidinedione-methylene)norketamine.

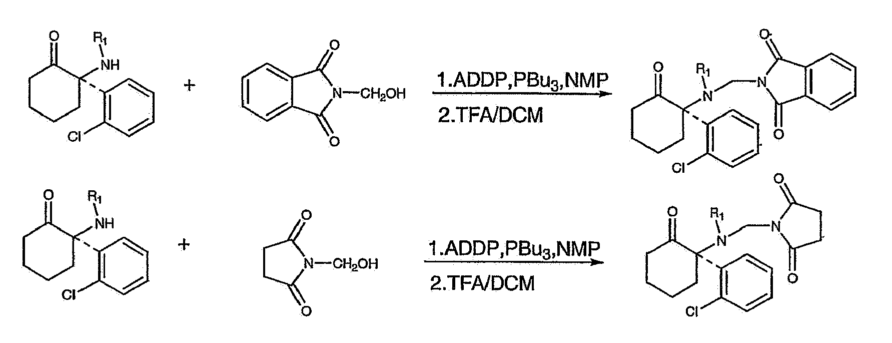

R₁=H or CH₃

These prodrugs are obtained from the Mitsunobu reaction between ketamine or norketamine and N-(hydroxymethyl)phthalimide or 1-(hydroxymethyl)-2,5-pyrrolidinedione, which are both commercially available (Zaragoza and Stephensen, 2000).

### 11. N-Phthalidoketamine and N-phthalidonorketamine

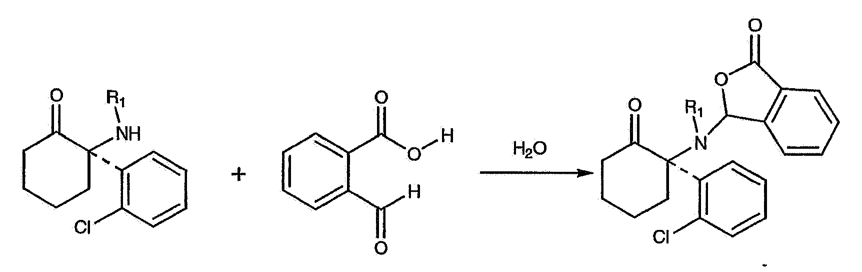

R₁=H or CH₃

Phthaladehylic acid (a commercial product) reacts with primary and secondary amines in aqueous solution to afford N-substituted phthalides (Wheeler, *et al,* 1957). N-phthalidoketamine and norketamine prodrugs can be prepared this way according to the scheme shown above.

### 12.N-(2-Oxo-1,3,dioxol-5-substituted-4-yl)methylketamines and N-(2-oxo-1,3,dioxol-5-substituted-4-yl)methylnorketamines.

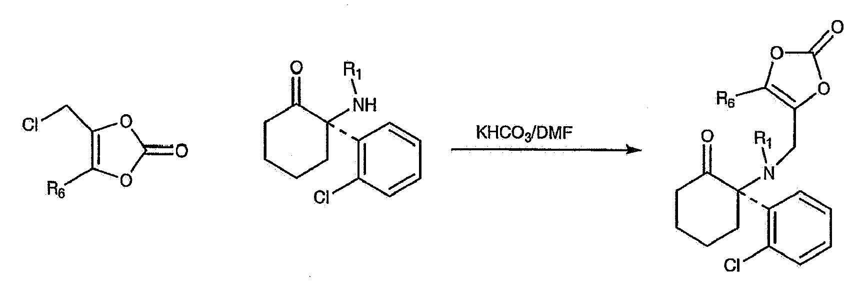

R₁=H or CH₃
R₆=alkyl, branched alkyl

These prodrugs are prepared from the reaction of an appropriate (2-oxo-1,3,dioxol-5-substituted-4-yl)methyl chloride (available from TESSENDERLO GROUP/COS, Belgium) with ketamine and norketamine (Kanebo, Ltd, 1983).

### Pulmonary And Nasal Transmucosal Administration Of Norketamine And Ketamine/Norketamine Prodrugs

The present invention contemplates formulations comprising norketamine and/or ketamine/norketamine prodrugs for use in a wide variety of devices that are designed for the delivery of pharmaceutical compositions and therapeutic formulations to the respiratory tract, preferably the pulmonary and bronchial passages. A preferred route of administration of the present invention is in an aerosol spray for pulmonary inhalation. Norketamine and/or ketamine/norketamine prodrugs, optionally combined with a dispersing agent, or dispersant, can be administered in an pulmonary formulation as a dry powder or in a solution or suspension, optionally with a diluent.

As used herein, the term "aerosol" refers to suspension in the air. In particular, aerosol refers to the particalization or atomization of a formulation of the invention and its suspension in the air. According to the present invention, a pulmonary formulation is a formulation comprising a norketamine and/or ketamine/norketamine prodrugs for inhalation or pulmonary administration.

As used herein, the term "inhaler" refers both to devices for nasal-transmucosal and pulmonary administration of a drug, e.g., in solution, powder and the like. For example, the term "inhaler" is intended to encompass a propellant driven inhaler or a dry powder inhaler, such as is used for to administer antihistamine for acute asthma attacks, and plastic spray bottles, such as are used to administer decongestants. As used herein, "inhaler" will also encompass the term "nebulizer" as it is well known in the art.

As used herein, the term "dispersant" refers to a agent that assists aerosolization or absorption of the norketamine and/or ketamine/norketamine prodrugs in mucosal tissue, or both. In a specific aspect, the dispersant can be a mucosal penetration enhancer. Preferably, the dispersant is pharmaceutically acceptable. As used herein, the term "pharmaceutically acceptable" means approved by a regulatory agency of the Federal or a state government or listed in the U.S. Pharmacopeia or other generally recognized pharmacopeia for use in animals, and more particularly in humans.

Suitable dispersing agents are well known in the art, and include but are not limited to surfactants and the like. For example, surfactants that are generally used in the art to reduce surface induced aggregation of norketamine or a ketamine/norketamine prodrug caused by atomization of the solution forming the liquid aerosol may be used. Nonlimiting examples of such surfactants are surfactants such as polyoxyethylene fatty acid esters and alcohols, and polyoxyethylene sorbitan fatty acid esters. Amounts of surfactants used will vary, being generally within the range or 0.001 and 4% by weight of the formulation. Suitable surfactants are well known in the art, and can be selected on the basis of desired properties, depending on the specific formulation, concentration of norketamine or a ketamine/norketamine prodrug, diluent (in a liquid formulation) or form of powder (in a dry powder formulation), etc.

The liquid formulations contain norketamine and/or ketamine/norketamine prodrugs, optionally with a dispersing agent, in a physiologically acceptable diluent. The dry powder formulations of the present invention consist of a finely divided solid form of norketamine and/or ketamine/norketamine prodrugs, optionally with a dispersing agent. With either the liquid or dry powder formulation, the formulation must be aerosolized. That is, it must be broken down into liquid or solid particles in order to ensure that the aerosolized dose actually reaches the mucous membranes of the bronchial passages or the lungs. The term "aerosol particle" is used herein to describe the liquid or solid particle suitable for transmucosal or pulmonary administration, *i.e.,* that will reach the mucous membranes or lungs. Other considerations, such as construction of the delivery device, additional components in the formulation, and particle composition and characteristics are important. These aspects of transmucosal or pulmonary administration of a drug are well known in the art, and manipulation of formulations, aerosolization means, and construction of a delivery device require, at most, routine experimentation by one of ordinary skill in the art.

For nasal or pulmonary administration, a useful device is a small, hard bottle to which a metered dose sprayer is attached. In one embodiment, the metered dose is delivered by drawing the norketamine and/or ketamine/norketamine prodrug solution into a chamber of defined volume, which chamber has an aperture dimensioned to aerosolize the formulation by forming a spray when a liquid in the chamber is compressed. The chamber is compressed to administer the norketamine and/or ketamine/norketamine prodrugs. In a specific embodiment, the chamber is a piston arrangement. Such devices are commercially available.

Alternatively, a plastic squeeze bottle with an aperture or opening dimensioned to aerosolize an pulmonary formulation by forming a spray when squeezed. The opening is usually found in the top of the bottle, and the top is generally tapered to partially fit in the nasal passages for efficient administration of the aerosol formulation. Preferably, the nasal or pulmonary inhaler will provide a metered amount of the formulation, for administration of a measured dose of the drug.

Often, the aerosolization of a liquid or a dry powder formulation for inhalation into the lung will require a propellent. The propellent may be any propellant generally used in the art. Specific nonlimiting examples of such useful propellants are a chloroflourocarbon, a hydrofluorocarbon, a hydochlorofluorocarbon, or a hydrocarbon, including trifluoromethane, dichlorodiflouromethane, dichlorotetrafluoroethanol, and 1,1,1,2-tetraflouroethane, or combinations thereof.

Systems of aerosol delivery, such as the pressurized metered dose inhaler and the dry powder inhaler are disclosed in Newman, S. P., Aerosols and the Lung, Clarke, S. W. and Davia, D. editors, pp. 197-222, and in U.S. Patent Nos. 6,358,530, 6,360,743, 6,406,745, 6,423,683, 6,565,888, and 6,630,169, the disclosures of which are incorporated herein in their entireties, and can be used in connection with the present invention.

In a further embodiment, as discussed in detail infra, a nasal transmucosal or pulmonary formulation of the present invention can include other therapeutically or pharmacologically active ingredients in addition to norketamine and/or ketamine/norketamine prodrugs, such as but not limited to a benzodiazepine or a narcotic analgesic.

In general, as described in detail infra, the norketamine and/or ketamine/norketamine prodrug is introduced into the subject in the aerosol form in an amount between about 0.01 mg per kg body weight of the mammal up to about 1 mg per kg body weight of said mammal. In a specific embodiment, the dosage is administered as needed. One of ordinary skill in the art can readily determine a volume or weight of aerosol corresponding to this dosage based on the concentration of norketamine and/or ketamine/norketamine prodrugs in a formulation of the invention.

In a particular embodiment, the mass median dynamic diameter will be 5 micrometers or less in order to ensure that the drug particles reach the lung alveoli (Wearley, L. L., 1991, 1991, Crit. Rev. in Ther. Drug Carrier Systems 8:333).

With regard to construction of the delivery device, any form of aerosolization known in the art, including but not limited to spray bottles, nebulization, atomization or pump aerosolization of a liquid formulation, and aerosolization of a dry powder formulation, can be used in the practice of the invention.

As noted above, in a preferred aspect of the invention, the device for aerosolization is a metered dose inhaler. A metered dose inhaler provides a specific dosage when administered, rather than a variable dose depending on administration. Such a metered dose inhaler can be used with either a liquid or a dry powder formulation. Metered dose inhalers are well known in the art.

### Transmucosal Administration Of Norketamine And Ketamine/Norketamine Prodrugs

As noted above, the present invention is directed inter alia to transmucosal administration of norketamine and/or ketamine/norketamine prodrugs. Initial studies demonstrate that nasal administration of norketamine and/or ketamine/norketamine prodrugs, either via the nasal mucosa or pulmonary inhalation and absorption via pulmonary mucosa, is highly effective for the treatment of pain. Subsequently, it has been discovered that other routes of transmucosal administration of norketamine and/or ketamine/norketamine prodrugs are also effective for treatment of pain, as set forth above. In particular, it has surprisingly been discovered that transmucosal administration of norketamine and/or ketamine/norketamine prodrugs allows for effective pharmacokinetics with low doses of the drug, thus avoiding dysphoria or other side effects associated with bolus i.v. or i.m. dosing. Transmucosal norketamine, and/or ketamine/norketamine prodrugs, is particularly indicated for breakthrough and spike pain, e.g., as described in greater detail above.

According to the invention, any transmucosal route of administration, including but not limited to rectal, oral, vaginal, buccal, etc. can be employed. In particular, the present invention is directed to the following transmucosal routes of administration. It can be readily appreciated that any of the transmucosal routes of administration may be enhanced by use of a mucosal penetration enhancer, *e.g*., as described supra. The selection of a particular mucosal penetration enhancer may depend on the characteristics of the specific mucosa. These factors are addressed in greater detail below.

### Administration Via Suppositories

In another aspect, norketamine and ketamine/norketamine prodrugs are formulated in a matrix suitable for rectal (or vaginal) insertion, *i.e.,* in a suppository. The invention is not limited to any particular suppository formulation. Indeed, many suppository formulations are known in the art, *e.g*., as described in Remington's Pharmaceutical Sciences, Physician's Desk Reference, and U.S. Pharmacopeia.

Administration via suppositories may be preferred in certain situations, *e.g*., because convention and custom prefers it, or where nasal administration is deemed unacceptable.

### Administration Via Buccal Patch

According to the invention, norketamine and/or ketamine/norketamine prodrugs can be formulated in a buccal patch for administration via the interior of the cheek. It may be appreciated that a buccal patch constitutes another form of transmucosal administration. The technology for preparing buccal patch formulations is known in the art, *e.g.,* Remington's Pharmaceutical Sciences, supra.

### Oral-Pharyngeal Administration

In yet another embodiment, norketamine and/or ketamine/norketamine prodrugs can be formulated for oral-pharyngeal, including sublingual and transbuccal, administration. For example, norketamine and/or ketamine/norketamine prodrugs can be incorporated in a "candy" matrix, such as that described in U.S. Pat. No. 4,671,953, in a gum base, or a lozenge. In another embodiment, the norketamine and/or ketamine/norketamine prodrugs can be formulated in a capsule or pill form for sublingual placement.

It is particularly contemplated that norketamine and/or ketamine/norketamine prodrugs for oral-pharyngeal administration may be formulated with a flavor masking agent or coating. Many flavor masking agents for use with oral pharmaceuticals are known in the art, and can be selected for use with the present invention.

### Oral Administration

In still a further embodiment, norketamine and/or ketamine/norketamine prodrugs can be formulated for oral administration via the stomach and intestinal mucosa. For oral administration, the drug can be administered in a carrier designed for drug release in either the stomach (an acidic environment), or the intestines, or both. Many capsules, pills, and matrices for oral administration of a drug are known in the art, and can be selected on the basis of compatibility with norketamine and ketamine/norketamine prodrugs, and the desired point and rate of drug release by the ordinary skilled physician. Sustained release formulations are preferred. In contrast to administration of ketamine, oral administration of norketamine and ketamine/norketamine prodrugs does not require higher dosages than other routes of administration to overcome the effects of first pass metabolism by the liver.

### Transdermal Administration

In a further embodiment, as noted above, the present invention is directed to transdermal administration of norketamine and/or ketamine/norketamine prodrugs. It has been discovered that transdermal administration is also effective for treatment of pain, as set forth above, for many of the same reasons transmucosal administration is effective. In particular, it has surprisingly been discovered that transdermal administration of norketamine and/or ketamine/norketamine prodrugs allows for effective pharmacokinetics with low doses of the drug, thus avoiding dysphoria or other side effects associated with bolus i.v. or i.m. dosing. Transdermal administration is particularly indicated for breakthrough and spike pain, *e.g.,* as described in greater detail above.

Various and numerous methods are known in the art for transdermal administration of a drug, *e.g.,* via a transdermal patch. These methods and associated devices provide for control of the rate and quantity of administration of a drug, and some allow for continuous modulation of drug delivery. Transdermal patches are described in, for example, U.S. Pat. No. 5,407,713, issued Apr. 18, 1995 to Rolando et al.; U.S. Pat. No. 5,352,456, issued Oct. 4, 1004 to Fallon *et al*.; U.S. Pat. No. 5,332,213 issued Aug. 9, 1994 to D'Angelo et al.; U.S. Pat. No. 5,336,168, issued Aug. 9, 1994 to Sibalis; U.S. Pat. No. 5,290,561, issued Mar. 1, 1994 to Farhadieh et al.; U.S. Pat. No. 5,254,346, issued Oct. 19, 1993 to Tucker et al.; U.S. Pat. No. 5,164,189, issued Nov. 17, 1992 to Berger et al.; U.S. Pat. No. 5,163,899, issued Nov. 17, 1992 to Sibalis; U.S. Pat. Nos. 5,088,977 and 5,087,240, both issued Feb. 18, 1992 to Sibalis; U.S. Pat. No. 5,008,110, issued Apr. 16, 1991 to Benecke et al.; and U.S. Pat. No. 4,921,475, issued May 1, 1990 to Sibalis, the disclosure of each of which is incorporated herein by reference in its entirety.

It can be readily appreciated that a transdermal route of administration may be enhanced by use of a dermal penetration enhancer, *e.g.,* such as enhancers described in U.S. Pat. No. 5,164,189 (supra), U.S. Pat. No. 5,008,110 (supra), and U.S. Pat. No. 4,879,119, issued Nov. 7, 1989 to Aruga *et al.,* the disclosure of each of which is incorporated herein by reference in its entirety.

### Additional Therapeutically Active Drugs Or Agents

As note above, the invention contemplates coordinate administration of norketamine and/or ketamine/norketamine prodrugs with a therapeutically effective amount of another drug, in particular a benzodiazepine or a narcotic analgesic.

Co-administration of the norketamine and/or ketamine/norketamine prodrugs with a benzodiazepine is indicated to counteract the potential dysphoric or hallucinogenic effects of high dose administration of norketamine and/or ketamine/norketamine prodrugs. Thus, a therapeutically effective amount of a benzodiazepine is an amount effective to inhibit dysphoria. In a further embodiment, an amount of a benzodiazepine also effective to sedate the patient may be administered.

The mild adverse effects of ketamine, *e.g.,* dysphoria and/or hallucinations, sometimes called "ketamine dreams," can occur upon administration of a dose of greater than 50 mg of ketamine, but usually require doses greater than 100 mg per kg of ketamine. One advantage of the present invention is that delivery of norketamine and/or ketamine/norketamine prodrugs allows for control of the dose to a level effective for analgesia, but below the level that results in dysphoria. Another is that norketamine and/or ketamine/norketamine prodrugs are less prone to adverse psychological effects than ketamine. However, it is possible that an individual may overdose, particularly in response to an acute episode of pain. Thus, co-administration of a benzodiazepine may be indicated in certain circumstances.

Benzodiazepines that may be administered according to the present invention include, but are not limited to, flurazepam (Dalmane), diazepam (Valium), and, preferably, Versed. In a preferred aspect, the transmucosal formulation of the invention comprises ketamine and a benzodiazepine, each present in a therapeutically effective amount.

In addition to the beneficial effects of norketamine and/or ketamine/norketamine prodrugs alone administered via a transmucosal, transdermal, or oral route, which are particularly effective for breakthrough or spike pain conditions, the present invention is directed to administration of the drugs via any route, including parenteral administration in addition to transmucosal, transdermal, and oral administration, in conjunction with a therapeutically effective amount of a narcotic analgesic used for the treatment of chronic pain. A therapeutically effective amount of a narcotic drug is an amount effective to alleviate pain. Such narcotics include, but are not limited to, fentanyl, meperidine (Demoral), morphine and its narcotic analogs and derivatives such as hydromorphine (Dilaudid), and the like. In a preferred aspect, the transmucosal formulation of the invention comprises norketamine and/or ketamine/norketamine prodrugs and a narcotic, each present in a therapeutically effective amount. Thus, the present invention is not limited to any particular mode or route of administration of norketamine or ketamine/norketamine prodrugs for their synergistic effects with other pain therapies, particularly drug administration, and most particularly, use of narcotic analgesics. Accordingly, where medical necessity or preference dictates, parenteral administration of norketamine and/or ketamine/norketamine prodrugs can be effected to synergistically treat pain with other pain therapies.

Alternate pain therapies include non-pharmaceutical treatments, such as but not limited to, chiropractic medicine, acupuncture, biofeedback, and other alternative therapies.

Preferably, the synergistic effects of norketamine and/or ketamine/norketamine prodrug administration are reflected by reduced dependency on other pain therapies, or by an reduction in the level of pain experienced, or both. This aspect of the invention is based on the surprising discovery administration of norketamine and/or ketamine/norketamine prodrugs allow for a reduction over time of narcotic analgesics. Such a reduction over time runs counter to the normal course of pain treatment, where progressively larger doses of analgesics, particularly narcotic analgesics, are required to overcome tolerance.

Usually, combinations of pain medications yield at best additive or supplemental results. Thus, it is a significant advantage of the present invention that it allows for a reduction in the level of a pain medication, without compromising the level of pain relief.

Parenteral administration generally refers to intravenous injection, and also includes, but is not limited to, intra-arteriole, intramuscular, intradermal, subcutaneous, intraperitoneal, intraventricular, and intracranial administration.

In another embodiment, the norketamine and/or ketamine/norketamine prodrugs can be delivered in a vesicle, in particular a liposome (see Langer, 1990, Science 249:1527-1533; Treat et al., 1989, in Liposomes in the Therapy of Infectious Disease and Cancer, Lopez-Berestein and Fidler (eds.), Liss: New York, pp. 353-365; Lopez-Berestein, ibid, pp. 317-327; see generally ibid). To reduce its systemic side effects, this may be a preferred method for introducing norketamine and/or ketamine/norketamine prodrugs.

In yet another embodiment, norketamine and/or ketamine/norketamine prodrugs may be delivered in a controlled release system. For example, the drugs may be administered using intravenous infusion, an implantable osmotic pump, a transdermal patch, liposomes, or other modes of sustained release administration. In one embodiment, a pump may be used (see Langer, supra; Sefton, 1987, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used (see Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, 1983, J. Macromol. Sci. Rev. Macromol. Chem. 23:61; see also Levy et al., 1985, Science 228:190; During et al., 1989, Ann. Neurol. 25:351; Howard et al., 1989, J. Neurosurg. 71:105).

Other controlled release systems are discussed in the review by Langer (1990, Science 249:1527-1533).

The present invention is not to be limited in scope by the specific embodiments describe herein. Indeed, various modifications of the invention in addition to those described herein will become apparent to those skilled in the art from the foregoing description and the accompanying figures. Such modifications are intended to fall within the scope of the appended claims.

### EXAMPLES

### Example 1

A female patient, age 40, weighing approximately 60 kg, presents with intractable bladder pain (interstitial ceptitis), which has been diagnosed previously. Pain management in this patient consists of 100 mg Demoral every 3 hours; Dilaudid 2-4 mg every 4 hours; Dalmane 30 mg per day; Duralgesic patches (fentanyl transdermal patches); bladder washes with Pyridium (phenaropyridine HCl), which is a urinary tract analgesic; and belladonna and opiate suppositories. In addition to the pain medication, the patient takes Zanax and Tagamet to alleviate gastric distress, and Compazine (an anti-emetic) to counteract nausea. Gastric distress and nausea in this patient result from the pain medication.

Despite the dosages and range of pain medications used by this patient, satisfactory pain management is not achieved.

A diagnostic pre-sacral, or ilio-hypogastric, nerve block is performed on this patient to alleviate the pain. Unfortunately, the effect of the block is temporary, and the block is associated with significant motor weakness. After the block wears off, the patient states that she is unable to function, as the most mundane activities are exhausting.

Norketamine (10 mg/cc) drip was administered i.v. over one hour, for a total dose of 40 mg norketamine. This results in reduction of the pain level by a factor of 2 (from #20 to about #10-12) as subjectively evaluated by the patient. About 1 hour after norketamine infusion is discontinued, the patient reports that the level of pain has increased to about #15, and thereafter rapidly to its previous level. The patient continues to take the other pain medications without effect.

Four days after the norketamine i.v. challenge, a 5 ml bottle containing 100 mg/ml norketamine solution is prepared. A single spray from the bottle delivers approximately 1/6 ml of solution, *i.e.,* 16 mg of norketamine. The patient is instructed to self-administer 1-2 sprays from the bottle for severe pain. The nasal spray bottle is prepared in order to provide sustainable pain medication on an outpatient basis.

The patient demonstrates remarkable pain management with nasal administration of norketamine. Nasal norketamine is particularly effective for control of breakthrough pain. The patient decreases the amount of the other pain medications.

### Example 2: Hydrolysis Study Protocol for Norketamine Prodrug

Stability studies are conducted both in Hanks' buffer of pH 7.4 and human plasma over a period of 48 hrs (n=3).

### Analytical Method:

From the stock solution of 1 mg/ml of norketamine esters and norketamine in acetonitrile, a series of standard solutions in the concentration range of 50-1000 ng/ml with acetonitrile are prepared. Hanks' buffer (300µL) and plasma (200µL) are spiked with 10µL of different concentrations of both the drug solutions. The Hanks' buffer samples are vortexed for 30 sec and centrifuged (20 min at 12000 rpm) and the supernatant is transferred to HPLC vials.

In the case of the plasma samples, 750µL of acetonitrile is added and vortexed for 30 sec and centrifuged (20 min at 12000 rpm) and the supernatant is removed. The supernatant is evaporated at 37° C under nitrogen and reconstituted with 400µL acetonitrile and transferred to HPLC vials. The HPLC system consists of a Perkin Elmer series 200 autosampler and pump and a 785A UV/VIS detector with Turbochrome 6.1 software. A reversed phase 220 x 4.6mm Brownlee Spheri 5 VL C-18 5µ column and a guard column are used. The detector is set at a wavelength of 215 nm. The mobile phase consistes of 0.1% trifuoroacetic acid (adjusted to pH 3 with triethylamine + 0.1% sodium heptane sulfonate and 5% acetonitrile):
acetonitrile (25:75) at a flow rate of 1.5 ml/min. Injection volume is 100µL and run time is 10 minutes.

Norketamine esters are hydrolyzed to norketamine in the plasma samples.

various publications are cited herein, the disclosures of which are incorporated by reference in their entireties.

While the invention has been described in connection with specific embodiments thereof, it will be understood that it is capable of further modifications and this application is intended to cover any variations, uses, or alterations of the invention following. In general, the principles of the invention and including such departures from the present disclosure as come within known or customary practice within the art to which the invention pertains and as may be applied to the essential features hereinbefore set forth and as follows in the scope of the appended claims.

## Claims

1. A compound of formula 1 or formula 2 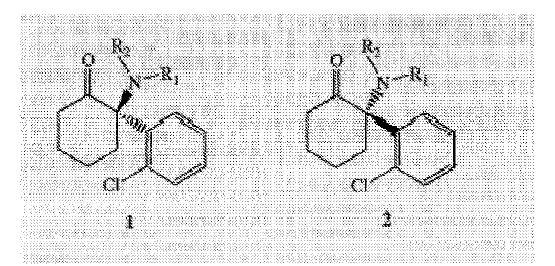 wherein :
R₁ = Methyl, R₂ = CH₂OCOR₃
R₁ = H, R₂ = CH₂OCOR₃
R₁ = Methyl, R₂ = CH₂COOR₃
R₁ = H, R₂ = CH₂COOR₃
R₁ = Methyl, R₂ = COOR₃
R₁ = H, R₂ = COOR₃
R₁ = Methyl, R₂ = COOCH₂CH₂N(CH3)₂
R₁ = H, R₂ = COOCH₂CH₂N(CH3)₂
R₁ = Methyl, R₂ = COOCH(R₃)OCOR₄
R₁ = H, R₂ = COOCH(R₃)OCOR₄ 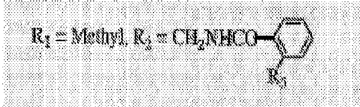 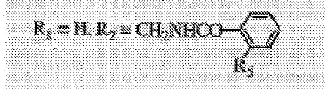 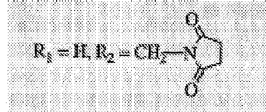 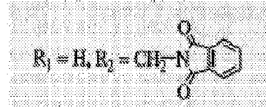 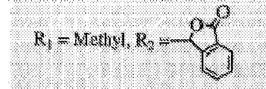 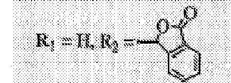 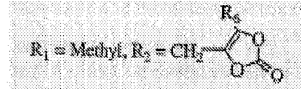 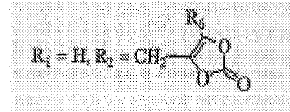
and wherein R₃ and R₄ are phenyl, aryl, azaaryl, alkyl, branched alkyl, cycloalkyl, alkenyl, cycloalkenyl ; where R₅ = OH or SH; and
where R₆ = alkyl, branched alkyl;
or a racemic mixture of compounds of formula 1 and formula 2 in which R₁ = H and R₂ can be any of the groups recited above for R₂, including H; and
pharmaceutical acceptable salts and solvates thereof.

2. The compound according to Claim 1, wherein the compound is:
[1-(2-Chloro-phenyl)-2-oxo-cyclohexyl]-carbamic acid ethyl ester;
[1-(2-Chloro-phenyl)-2-oxo-cyclohexyl]-carbamic acid isopropyl ester;
[1-(2-Chloro-phenyl)-2-oxo-cyclohexyl]-carbamic acid butyl ester;
[1-(2-Chloro-phenyl)-2-oxo-cyclohexyl]-carbamic acid phenyl ester;
[1-(2-Chloro-phenyl)-2-oxo-cyclohexyl]-carbamic acid benzyl ester;
[1-(2-Chloro-phenyl)-2-oxo-cyclohexylamino]-acetic acid ethyl ester;
or any combination thereof, or any pharmaceutical acceptable salts or solvates thereof.

3. Drug composition comprising a compound according to Claim 1 or Claim 2.

4. Drug composition according to Claim 3, wherein the compound is (±)norketamine, S-norketamine, R-norketamine, or any combination thereof, or any pharmaceutically acceptable salts or solvates thereof.

5. Drug composition according to Claim 3 or Claim 4, wherein the compound is a prodrug of (±)norketamine, a prodrug of (±) ketamine, a prodrug of S-ketamine, a prodrug of R-ketamine, a prodrug of S-norketamine, or a prodrug of R-norketamine, or any combination thereof, or any pharmaceutical acceptable salts or solvates thereof.

6. Drug composition according to any one of Claims 3 - 5, further comprising a benzodiazepine, a narcotic analgesic or a combination thereof.

7. Use of a compound according to Claim 1 or Claim 2 for the manufacture of a medicament for the treatment of pain in a subject.

8. Use according to Claim 7, wherein the pain is breakthrough pain, pain associated with wind-up, pain associated with labour and/or childbirth, chronic pain or neuropathic pain.

9. Use according to Claim 6 or Claim 7, wherein the medicament can be self-administered via one or more of the transmucosal, transdermal, nasal, oral, or pulmonary routes, or any combination thereof.

10. A device for patient self-administration of a compound of Claim 1 or Claim 2 on an outpatient basis comprising a nasal applicator containing a formulation of the compound according to Claim 1 or Claim 2 and a pharmaceutically acceptable vehicle, wherein the device is metered to disperse an amount of the formulation that contains a dose of the compound.

11. The device according to Claim 10, wherein the compound is (±)norketamine, S-norketamine, R-norketamine, or any combination thereof, or any pharmaceutically acceptable salts or solvates thereof.

12. The device according to Claim 10 or Claim 11, wherein the compound is a prodrug of (±)norketamine, a prodrug of (±)ketamine, a prodrug of S-ketamine, a prodrug of R-ketamine, a prodrug of S-norketamine, or a prodrug of R-norketamine, or any combination thereof, or any pharmaceutically acceptable salts or solvates thereof.

13. The device according to any one of Claims 10 - 12, wherein the compound is:
[1-(2-Chloro-phenyl)-2-oxo-cyclohexyl]-carbamic acid ethyl ester;
[1- (2-Chloro-phenyl)-2-oxo-cyclohexyl]-carbamic acid isopropyl ester;
[1- (2-Chloro-phenyl)-2-oxo-cyclohexyl]-carbamic acid butyl ester;
[1- (2-Chloro-phenyl)-2-oxo-cyclohexyl]-carbamic acid phenyl ester;
[1- (2-Chloro-phenyl)-2-oxo-cyclohexyl]-carbamic acid benzyl ester;
[1-(2-Chloro-phenyl)₋2-oxo-cyclohexylamino]-acetic acid ethyl ester;
or any combination thereof, or any pharmaceutical acceptable salts or solvates thereof.

14. The device according to any one of Claims 10 - 13, wherein the formulation further comprises a benzodiazepine, a narcotic analgesic or a combination thereof.

15. The device according to any one of Claims 10 - 14, wherein the formulation is a sustained-release formulation.
